(19) Europäisches Patentamt
European Patent Office
Office européen des brevets

(11) EP 2 011 498 A1

(12) **EUROPEAN PATENT APPLICATION**
published in accordance with Art. 153(4) EPC

(43) Date of publication:
**07.01.2009 Bulletin 2009/02**

(21) Application number: **07740307.9**

(22) Date of filing: **29.03.2007**

(51) Int Cl.:
*A61K 31/4745* (2006.01) *A23L 1/30* (2006.01)
*A61K 31/122* (2006.01) *A61P 25/28* (2006.01)
*A61P 43/00* (2006.01) *C07D 471/04* (2006.01)

(86) International application number:
**PCT/JP2007/056869**

(87) International publication number:
**WO 2007/119588 (25.10.2007 Gazette 2007/43)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **10.04.2006 JP 2006107928**

(71) Applicant: **Mitsubishi Gas Chemical Company, Inc.**
**Chiyoda-ku**
**Tokyo 100-8324 (JP)**

(72) Inventors:
- **KIKKAWA, Kazutoshi**
  **Tokyo 100-8324 (JP)**
- **NAKANO, Masahiko**
  **Niigata-shi, Niigata 950-3112 (JP)**
- **URANO, Shiro**
  **Kawagoe-shi Saitama 350-1108 (JP)**

(74) Representative: **Harrison, Robert John et al**
**24IP Law Group Sonnenberg Fortmann**
**Patent- & Rechtsanwälte**
**Postfach 33 08 65**
**80068 München (DE)**

# (54) BRAIN FUNCTION-IMPROVING AGENT, AND FUNCTIONAL FOOD CONTAINING THE IMPROVING AGENT

(57) There are provided a brain function improving agent There are provided a brain function improving agent having an effect of improving learning and memory abilities, and a functional food containing the function improving agent. A preparation comprising a pyrroloquinoline quinone or a salt thereof alone or in combination with coenzyme Q10 and a functional food containing the preparation can improve learning and memory abilities decreased due to brain function disorders caused by various factors.

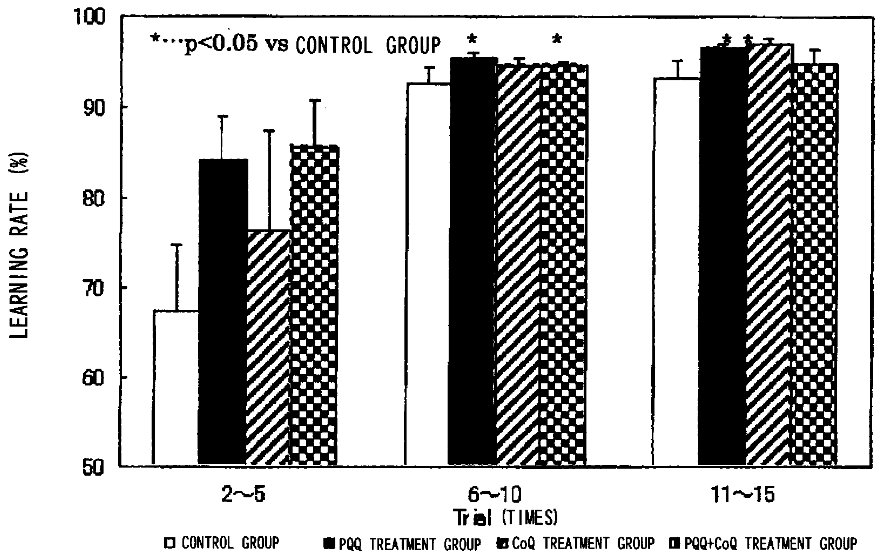


Figure 1 MEAN LEARNING RATES IN LEARNING TEST

EP 2 011 498 A1

## Description

### TECHNICAL FIELD

The present invention relates to a brain function improving agent comprising, as an active ingredient, at least a pyrroloquinoline quinone represented by the general formula (1) or a salt thereof, and a functional food containing the improving agent. Furthermore, the present invention relates to a brain function improving agent comprising, as active ingredients, coenzyme Q10 in combination with a pyrroloquinoline quinone represented by the general formula (1) or a salt thereof, and a functional food containing the improving agent. Such brain function improving agents and functional foods are very useful in improvement of decreased brain functions caused by vascular or organic damages because they have effects of improving decreased learning and memory abilities.

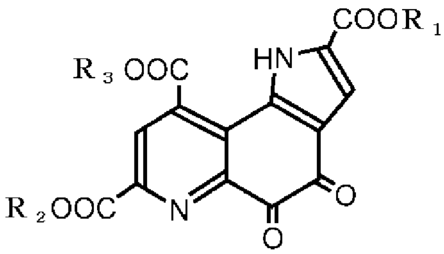

(1)

In the formula, $R_1$, $R_2$, and $R_3$ are identical or different, and represent a hydrogen atom, an alkyl group, an alkenyl group, a benzyl group, a propagyl group, or an alkoxycarbonylalkyl group.

### BACKGROUND ART

In recent years, the percentage of the elderly in the total population is rapidly increasing. Along with this increase, decrease in learning and memory abilities with aging and increase in dementia due to diseases have become problems. Diseases causing dementia include degenerative diseases of the brain represented by Alzheimer's disease, cerebrovascular disorders due to cerebral infarction and cerebral hemorrhage, brain tumor, head trauma, infectious diseases, metabolic diseases, and so forth. In particular, the number of patients with Alzheimer's disease has markedly increased with the advancement of aging society. It is said that patients with Alzheimer's disease accounts for about 30% of patients with dementia in Japan and 40 to 60% in the U.S. This disease is characterized by acute disturbance of short-term memory or remote memory, personality disorder and so forth, and has led to serious social problems from standpoints of medical expenses and nursing care.

This disease is associated with atrophy, loss, and the like of brain tissues as well as decreases in levels of acetylcholine which is a neurotransmitter. Characteristics of this disease further include senile plaques and neurofibrillary tangles observed in cerebral cortex and hippocampus, and its etiological mechanism is being studied on both amyloid β proteins which exist in the center of senile plaques and tau proteins which are structural proteins of neurofibrillary tangles. Development of therapeutic agents has been advanced based on such etiological mechanisms, and acetylcholine esterase inhibitors and the like have been developed. However, they cannot be said to be successful so far. In other words, recovery after onset is usually difficult, and current therapies have to rely upon delaying progression of the disease like symptomatic treatment.

Meanwhile, with the recent advancement in the research on functions of food ingredients, ingredients that have effects on brain functions have been found, and functions of preventing this disease and suppressing progression of the disease are actively explored in foods (for example, refer to Patent Documents 1 to 3). Docosahexaenoic acid (DHA), Ginkgo biloba extract, and the like have been known as food materials that activate brain functions. However, their effects are not adequate, and development of more effective food materials is demanded.

Patent Document 1: Japanese Patent Laid-Open (Kokai) No. H07-017855.

Patent Document 2: Japanese Patent Laid-Open (Kokai) No. H07-143862.

Patent Document 3: Japanese Patent No. 3195594.

## DISCLOSURE OF THE INVENTION

## PROBLEMS TO BE SOLVED BY THE INVENTION

An object of the present invention is to provide a preparation having an effect of improving brain functions damaged by vascular or nonvascular causes, and a functional food containing such a preparation.

## MEANS FOR SOLVING THE PROBLEM

The present inventors have conducted various researches on bioactivity of pyrroloquinoline quinones or salts thereof. As a result, they have found that oral dosing of these compounds has effects of markedly improving learning and memory abilities of laboratory animals, and further a combined use thereof with coenzyme Q10 exhibits a synergetic effect. Thus, the present invention has been accomplished.

Specifically, as described in the following items 1 to 4, the present invention relates to a preparation and a functional food, which have a brain function improving effect such as improvement of decreased learning and memory abilities and are characterized by comprising a pyrroloquinoline quinone or a salt thereof and, if desired, coenzyme Q10. Specifically, the present invention relates to a brain function improving agent that improves decreased learning and memory abilities due to aging and dementia such as Alzheimer's disease, and a functional food containing the improving agent.

1. A brain function improving agent comprising at least a pyrroloquinoline quinone represented by the general formula (1) or a salt thereof:

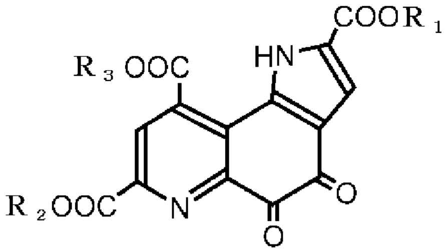

(1)

wherein $R_1$, $R_2$, and $R_3$ are identical or different, and represent a hydrogen atom, an alkyl group, an alkenyl group, a benzyl group, a propagyl group, or an alkoxycarbonylalkyl group.

2. The brain function improving agent according to the above item 1, further comprising coenzyme Q10.

3. The brain function improving agent according to the above item 1 or 2, which is useful for improving decreased learning and memory abilities.

4. A functional food comprising the brain function improving agent according to any one of the above items 1 to 3.

## EFFECTS OF THE INVENTION

The present invention relates to a brain function improving agent that comprises, as an active ingredient, a pyrroloquinoline quinone or a salt thereof, or a pyrroloquinoline quinone or a salt thereof together with coenzyme Q10, and has a brain function improving effects such as improvement of decreased learning and memory abilities, and relates to a functional food containing the function improving agent. Specifically, the present invention relates to a brain function improving agent that improves decrease in learning and memory abilities due to aging and dementia such as Alzheimer's disease, and a functional food containing the improving agent.

## BEST MODE FOR CARRYING OUT THE INVENTION

The present invention relates to a brain function improving agent comprising, as an active ingredients, a pyrroloquinoline quinone or a salt thereof, or a pyrroloquinoline quinone or a salt thereof in combination with coenzyme Q10, and a functional food containing the improving agent.

Pyrroloquinoline quinones were discovered in 1979 as coenzymes of methanol dehydrogenase in methanol assimilating bacteria. In addition to bacteria, they have also been detected in dietary plants such as soybean, broad bean, green pepper, potato, parsley, and spinach and processed foods such as vinegar, tea, cocoa, natto, and tofu.

Pyrroloquinoline quinones and salts thereof can be produced by organic chemical synthesis methods (for example, JACS, Vol. 103, pp. 5599-5600 (1981)), fermentation (for example, Japanese Patent Laid-Open (Kokai) No. H01-218597), or the like.

Examples of pyrroloquinoline quinones and salts thereof include pyrroloquinoline quinones represented by the general formula (1) and salts thereof which may be exemplified by alkali metal salts such as sodium salts and potassium salts, and alkaline earth metal salts such as magnesium salts and calcium salts but are not limited thereto.

Conventionally, pyrroloquinoline quinones and salts thereof have been evaluated for the purpose of medical use by intravenous infusion or intraperitoneal administration. However, these administration routes are not convenient but have serious problems such that they are invasive and liable to cause adverse drug reactions. Under the circumstances, their effects have not yet been exhibited without adverse drug reactions, and their efficacy has not yet been properly evaluated. However, the present inventors have unexpectedly found that oral ingestion of these compounds exhibits brain function improving effects such as improvement of decreased learning and memory abilities in a very small ingestion amount thereof, and the effective range of ingestion amount thereof is very wide whilst no adverse drug reaction occurs even in a high ingestion amount thereof. These findings suggest that pyrroloquinoline quinones and salts thereof can be used as safe materials for functional foods.

The pyrroloquinoline quinone represented by the general formula (1) or a salt thereof is used alone or in combination with coenzyme Q10, and can also be used in combination with other functional food materials. Examples of functional food materials that can be used in combination include L-carnitine, α-lipoic acid, vitamins used as food such as vitamin B family, vitamin C, and vitamin E, amino acids, carotenoids such as astaxanthin, α-carotene and β-carotene, ω3 fatty acids such as docosahexaenoic acid and eicosapentaenoic acid, ω6 fatty acids such as arachidonic acid, and so forth, but are not limited to these examples.

Furthermore, the present brain function improving agent comprising a pyrroloquinoline quinone represented by the general formula (1) or a salt thereof and optionally coenzyme Q10 can be used in a form of food and drink including drinks, syrups, various hospital diets, nutritional supplements, and food and drink on ordinary diet as well as tablets, capsules, and granules. Examples of additives that can be used as liquid agents in the preparation include water, saccharides such as fructose and glucose, oils such as peanut oil, soybean oil, and olive oil, and glycols such as polyethylene glycol and polypropylene glycol. Examples of excipients for solid preparations such as tablets, capsules, and granules include lactose, sucrose, and mannitol. Examples of lubricants include kaolin, talc, and magnesium stearate. Examples of disintegrating agents include starch and sodium alginate. Examples of binders include polyvinyl alcohol, cellulose, and gelatin. Examples of surfactants include fatty acid esters. Examples of plasticizers include glycerine and the like. However, examples are not limited to the above examples.

## EXAMPLES

Hereinafter, the present invention will be explained more specifically by way of the following examples. However, the present invention is not limited to these examples.

### Example 1

Male Wistar rats (9 weeks old) were bred using a feed containing 0.02% pyrroloquinoline quinone disodium salt (PQQ·2Na) or 0.3% coenzyme Q10 (CoQ10) alone or both in combination or a feed free from these compounds. From the age of 12 week old after 21 days of breeding, 9 animals per group were subjected to a test of learning and memory abilities using a Morris water maze. Specifically, the bottom of a circular pool (150 cm in diameter, 45 cm in depth) was divided into four quadrants with white lines. A circular platform was placed at the center of one of the quadrants so that it was hidden under the water. Each rat was allowed to swim from different positions of the other three quadrants having no platform, and the time until it reached the platform was measured. This test was tried once daily for a total of 20 days. The learning rate was calculated by the following equation:

$$\text{Learning rate (\%)} = 100 - (\text{mean time until rat reaches the platform at the Xth trial/mean time until rat reaches the platform at the first trial}) \times 100$$

In the above-mentioned water maze test, the learning rate increased with the increase in the number of trials in both the PQQ·2Na-added treatment groups and the PQQ·2Na-free treatment groups. However, the increasing rate

of the PQQ·2Na-added treatment groups was statistically significantly higher, and thus it was confirmed that PQQ·2Na improved memory and learning abilities. The results are shown in Figure 1.

Example 2

Male Wistar rats (9 weeks old) were bred using a feed containing 0.02% PQQ·2Na or 0.3% CoQ10 alone or both in combination or a feed free from these compounds. From the age of 12 week old after 21 days of breeding, 9 animals per group were allowed to memorize the position of the platform by the same training using the Morris water maze as in Example 1. The bottom of the pool was divided into four quadrants with white lines. A platform was placed at the center of one of the quadrants. The animals which had memorized the position of the platform were exposed to oxidative stress (under 100% oxygen for 48 hours). Then, the animals were allowed to swim for 60 seconds in the water maze from which the platform had been removed, and the duration of staying in the quadrant where the platform had been placed was measured. The memory retention rate was calculated by the following equation:

Memory retention rate (%) = (mean staying time over 9 days after exposure to oxidative stress/mean staying time immediately before exposure to oxidative stress) x 100.

The results are shown in Figure 2. The mean memory retention rate was highest in the group treated with PQQ·2Na plus CoQ10, followed by the group treated PQQ·2Na alone. Furthermore, a significant synergistic effect was observed in the mean memory retention rate when PQQ·2Na and CoQ10 were used in combination.

BRIEF DESCRIPTION OF THE DRAWINGS

Figure 1 is a graph showing effects on the rate of improvement of memory and learning abilities observed in Example 1; and

Figure 2 is a graph showing effects on the memory retention ability observed in Example 2.

**Claims**

1. A brain function improving agent comprising at least a pyrroloquinoline quinone represented by the following general formula (1) or a salt thereof:

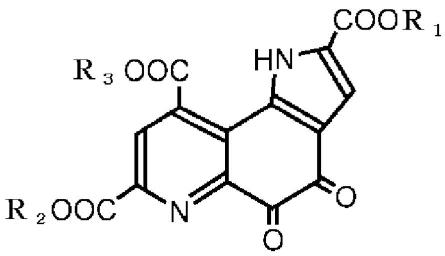

(1)

wherein $R_1$, $R_2$, and $R_3$ are identical or different, and represent a hydrogen atom, an alkyl group, an alkenyl group, a benzyl group, a propagyl group, or an alkoxycarbonylalkyl group.

2. The brain function improving agent according to claim 1, further comprising coenzyme Q10.

3. The brain function improving agent according to claim 1 or 2, which is useful for improving decreased learning and memory abilities.

4. A functional food comprising the brain function improving agent according to any one of claims 1 to 3.

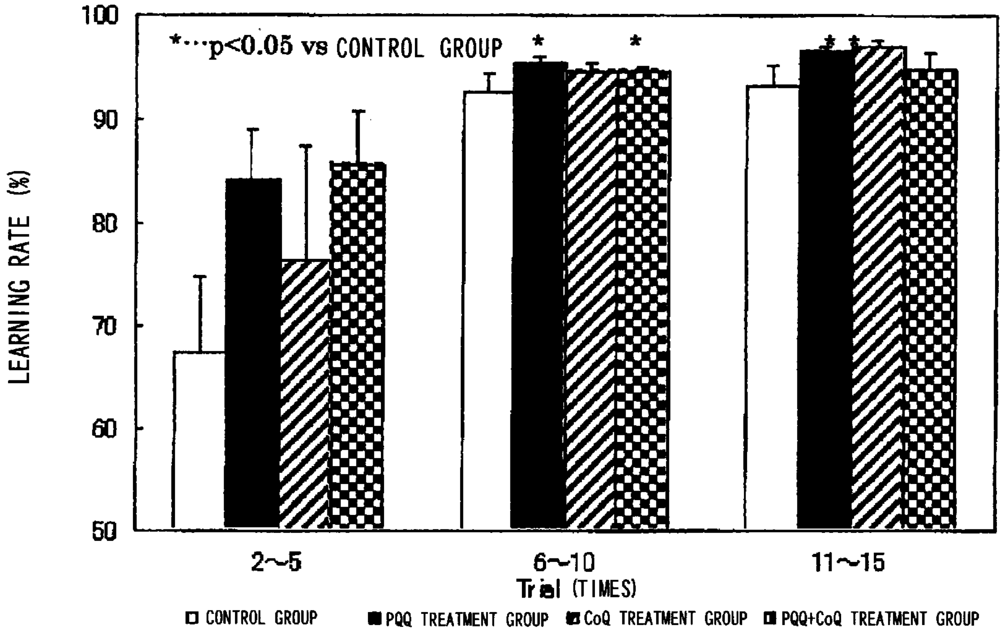


Figure 1 MEAN LEARNING RATES IN LEARNING TEST

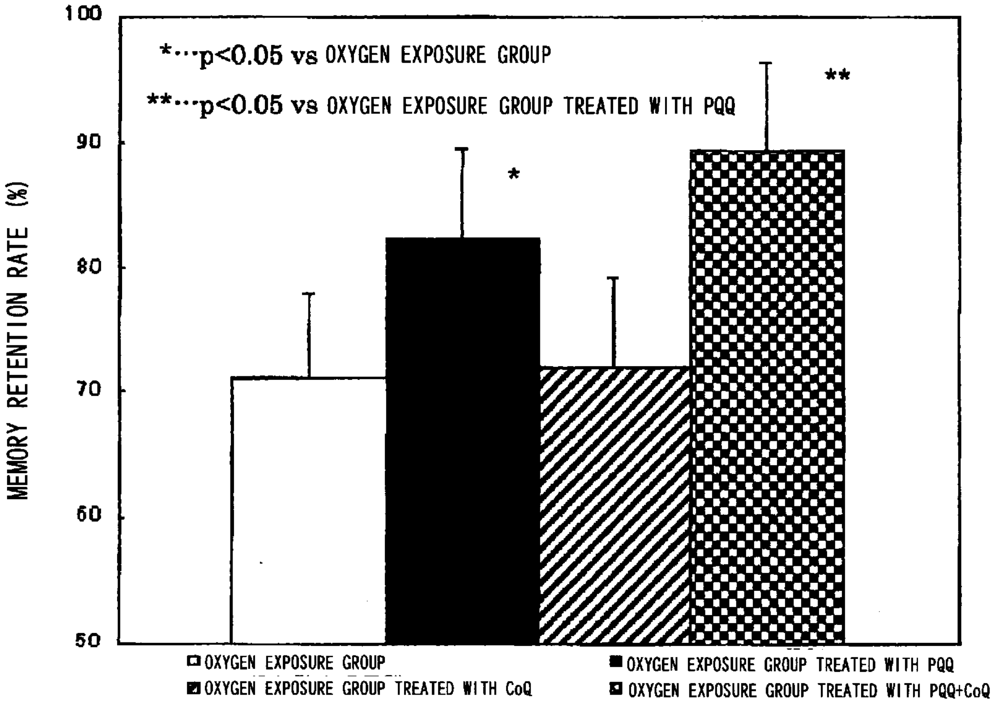


Figure 2 MEAN MEMORY RETENTION RATES IN MEMORY TEST

**INTERNATIONAL SEARCH REPORT**

International application No.
PCT/JP2007/056869

A. CLASSIFICATION OF SUBJECT MATTER
*A61K31/4745*(2006.01)i, *A23L1/30*(2006.01)i, *A61K31/122*(2006.01)i, *A61P25/28*(2006.01)i, *A61P43/00*(2006.01)i, *C07D471/04*(2006.01)i

According to International Patent Classification (IPC) or to both national classification and IPC

B. FIELDS SEARCHED

Minimum documentation searched (classification system followed by classification symbols)
A61K31/4745, A23L1/30, A61K31/122, A61P25/28, A61P43/00, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2-196720 A (Fuji Chemical Industry Co., Ltd.),<br>03 August, 1990 (03.08.90),<br>Claims; page 2, upper right column, lines 1 to 5; examples<br>(Family: none) | 1,3,4<br>2 |
| X<br>Y | JP 2-262581 A (Fuji Chemical Industry Co., Ltd.),<br>25 October, 1990 (25.10.90),<br>Claims; examples<br>(Family: none) | 1,3,4<br>2 |
| X<br>Y | JP 2005-82523 A (Toru HASEGAWA),<br>31 March, 2005 (31.03.05),<br>Full text<br>(Family: none) | 1,3,4<br>2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 June, 2007 (14.06.07) | Date of mailing of the international search report<br>26 June, 2007 (26.06.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2007/056869

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 6-211660 A (Sagami Chemical Research Center),<br>02 August, 1994 (02.08.94),<br>Claims; Par. Nos. [0001], [0006]; examples<br>& US 5589481 A & US 5846977 A<br>& EP 555149 A1 | 1,3,4<br>2 |
| Y | JP 2006-69958 A (Baio Igaku Kenkusho Kabushiki Kaisha),<br>16 March, 2006 (16.03.06),<br>Claims; Par. No. [0006]; examples<br>(Family: none) | 2 |
| Y | JP 2002-541216 A (National Research Council of Canada),<br>03 December, 2002 (03.12.02),<br>Par. No. [0037]<br>& WO 2000/061189 A2 & US 6045826 A<br>& EP 1159006 A2 & FI 20011914 A<br>& CA 2369300 A | 2 |
| Y | JP 61-33118 A (Seuref AG.),<br>17 February, 1986 (17.02.86),<br>Claims; page 2, upper right column, lines 16 to 20; page 4, upper left column, line 17 to lower right column, the last line<br>& US 4684520 A & GB 2157171 A<br>& FR 2562422 A & DE 3417857 A | 2 |
| Y | JP 2005-325086 A (Asahi Kasei Fama Kabushiki Kaisha),<br>24 November, 2005 (24.11.05),<br>Par. No. [0004]<br>(Family: none) | 2 |
| Y | WO 2005/089740 A1 (Kaneka Corp.),<br>29 September, 2005 (29.09.05),<br>Claims; Par. Nos. [0004], [0025]<br>(Family: none) | 2 |
| Y | JP 2003-26567 A (Kaneka Corp.),<br>29 January, 2003 (29.01.03),<br>Par. No. [0003]<br>& WO 2002/092067 A1 & US 2004/0115181 A1<br>& EP 1388340 A1 & CA 2443191 A<br>& CN 1505505 A | 2 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H07017855 B
- JP H07143862 B
- JP 3195594 B
- JP H01218597 B


### Non-patent literature cited in the description


- *JACS,* 1981, vol. 103, 5599-5600